# EUROPEAN PATENT APPLICATION

(11) **EP 1 306 102 A1**
(43) Date of publication of application: **02.05.2003**
(21) Application number: 02102478.1
(22) Date of filing: 23.10.2002
(51) Int. Cl.: A61M 36/12, A61M 5/32

(54) **IMPLANTER HAVING NEEDLE PROTECTION DEVICE**

(30) Priority: 24.10.2001 GB 1256114
(71) Applicant: Sterimatic Holdings Limited, Road Town Tortola British Virgin Islands (VG)
(72) Inventor: Rowley, Paul, c/o Sterimatic Holdings ltd., Tortola (VG)
(74) Representative: Harding, Richard Patrick

(57) **Abstract**

An implanter (50) for introducing an implant (56) under the skin of a human or animal body comprises a needle holder (53) for a hollow needle (54) having a tip (55) within which the implant (56) is accommodated and by means of which the implant (56) is introduced under the skin. The implanter (50) also comprises a plunger (57) for relative movement within the needle (54), and a depth guide (61) for contacting the surface of the skin during introduction of the needle tip (55) under the skin. Appropriate movement between the needle (54) and the plunger (57) when the needle tip (55) has been introduced under the skin to a required depth causes the implant (56) to be left at the required depth when the needle tip (55) is withdrawn. The implanter (50) incorporates a needle tip protection sleeve (70) for shielding the needle tip (55) prior to use, and a guide passage (72) for enabling the implant (56) to be loaded within the needle tip (55) whilst the needle tip (55) is shielded by the needle tip protection sleeve (70). Such an implanter (50) allows the implant (56) to be safely loaded within the implanter and to be located accurately within the body, and avoids any danger of the user being injured by the needle of the implanter.

## Description

This invention relates to implanters for introducing implants into a human or animal body.

There are a number of applications in which it is required to introduce an implant under the skin of a human or animal. For example it is a common practice to provide a domestic animal with an identity tag in the form of an electronic implant which is introduced under the animal's skin and which can be subsequently detected by a scanner externally of the animal's body in order to ascertain the animal's identity and possibly to retrieve other data relating to the animal. A similar implant may also be used as a carrier to enable slow release of a chemical or hormone within the body.

However a number of difficulties have been encountered in the introduction of such electronic implants into an animal's body. Because conventional implanters for introducing such implants into an animal's body simply comprise a needle having a hollow tip for accommodating the implant, and a plunger which can be operated to push the implant out beyond the needle tip into the animal's body when the needle tip has been introduced under the animal's skin, it is not possible to accurately control the position in which the implant is left in the animal's body. This can result in migration of the implant within the animal's body making it difficult to subsequently locate the implant and possibly causing medical problems as a result of such migration. Furthermore, since the implant is only loosely held within the needle tip, the implant can easily fall out of the needle tip and be lost prior to introduction into the animal's body. There is also a danger that the user will be accidentally pierced by the needle tip after it has been used to place the implant in the animal's body, and this can result in infection and transmission of blood borne diseases.

EP 0858813A2 discloses an implanter which incorporates a depth guide for contacting the surface of the skin during introduction of the needle tip under the skin and for effecting relative movement between the needle and the plunger when the needle tip has been introduced under the skin to the required depth in order to cause the implant to be left at the required depth when the needle tip is withdrawn. Such an implant allows the implant to be located accurately within the body and thus avoids the problems associated with incorrect placing of the implant, as well as substantially removing the risk of the implant falling out of the needle tip and being lost before it can be introduced into the body. However there is a possibility that the user may be injured by the needle when loading the implant into the needle tip prior to placement in the animal's body.

It is an object of the invention to provide an improved implanter for introducing implants into human or animal bodies.

According to the present invention there is provided an implanter for introducing an implant under the skin of a human or animal body, the implanter comprising a needle holder for a hollow needle having a tip within which the implant is accommodated and by means of which the implant is introduced under the skin, and a plunger for relative movement within the needle, characterised in that the implanter incorporates needle tip protection means for shielding the needle tip prior to use and having guide means for enabling the implant to be loaded within the needle tip whilst the needle tip is shielded by the needle tip protection means.

Such an arrangement is advantageous because it enables the implant to be loaded into the needle tip in a secure manner without any danger of the user being injured by the needle. This enables the implant to be held more positively within the needle tip, thus substantially removing the risk of the implant falling out of the needle tip and being lost before it has been introduced into the body. This is particularly advantageous in the case of electronic tag implants as such implants are costly and are small enough to be easily lost. The design of the implanter may also be such as to effectively remove the risk of the implant being damaged during placement, or of additional components being left behind within the body.

The needle tip protection means may incorporate a portion which extends beyond the needle tip when in a shielding position, and the guide means may comprise a guide passage in said portion through which the implant is loadable. Furthermore the needle tip protection means is preferably detachable from the implanter to enable the implant to be introduced under the skin by the needle.

The needle tip protection means preferably comprises sleeve means which surrounds at least an end portion of the needle when in a shielding position. In this case the sleeve means conveniently has an inner bore for receiving the needle having an end part which is adapted to the shape of the needle tip to ensure a required relative orientation between the sleeve means and the needle.

It is also advantageous for the implanter to be provided with a loading member for pushing the implant into the needle tip by way of the guide means.

Preferably a depth guide is provided for contacting the surface of the skin during introduction of the needle tip under the skin and for effecting relative movement between the needle and the plunger when the needle tip has been introduced under the skin to a required depth in order to cause the implant to be left at the required depth when the needle tip is withdrawn. Because the implant is not pushed out beyond the needle tip but instead is caused to be left behind at the required depth, such an implanter allows the implant to be located accurately within the body and thus avoids the problems associated with incorrect placing of the implant.

In a preferred embodiment of the invention needle shielding means is provided for shielding the needle tip when it has been withdrawn from the skin. For example, the needle shielding means may be constituted by a part of the depth guide into which the needle tip is retractable after completion of implantation.

Furthermore locking means may be provided to hold the needle in a retracted position in which the tip of the needle is shielded by the shielding means and accidental exposure of the needle tip is prevented.

In one embodiment the locking means comprises a latch which serves to couple together the shielding means and the needle holder in order to prevent relative movement between the needle and the shielding means in such a manner as to expose the needle tip.

In another embodiment the locking means comprises a resilient finger which is overridden by the needle tip when it is retracted into the needle shielding means and which prevents accidental exposure of the needle tip.

The plunger is advantageously provided with an extension which holds the implant within the needle tip until it is expelled by the plunger. For example, the extension may comprise a thin tailpiece which projects for the end of the plunger so as to hold the implant between the tailpiece and an inside wall of the needle.

Furthermore the implanter conveniently incorporates a substantially cylindrical handgrip from which the needle axially extends and which is adapted to be grasped by the user during introduction of the implant.

In a variant embodiment of the invention a spring may be provided to effect automatic retraction of the needle relative to the plunger to expel the implant from the needle when the needle tip has been introduced under the skin to the required depth. In this case the depth guide may be adapted to be axially displaced by contact with the skin in order to release a coupling between the needle and the plunger so as to permit retraction of the needle by the spring.

In order that the invention may be more fully understood, an implanter in accordance with the invention will now be described, by way of example, with reference to the accompanying drawings, in which:
Figure 1 is an axial section through the implanter;
Figure 2 is a cross-section taken along the line A-A in Figure 1;
Figure 3 is an axial section through an end of a needle protector of the implanter; and
Figures 4 to 9 are views of the implanter in successive operational modes during loading of the implant and introduction of the implant under the skin.

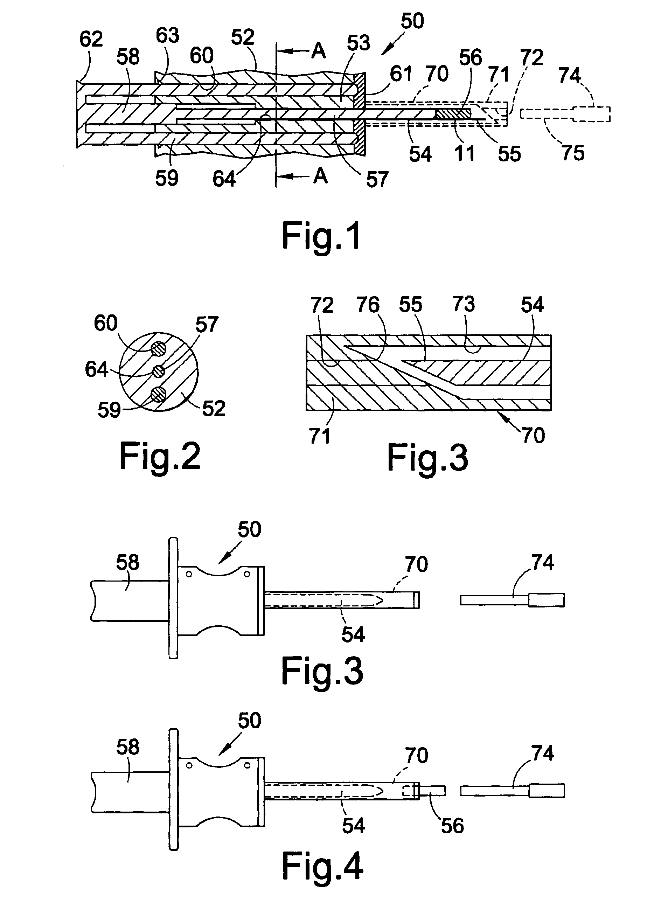

Figure 1 shows an implanter 50 comprising a generally cylindrical hollow handgrip 52 having an outer surface which is adapted to be grasped by the user's hand and accommodating therein a needle holder 53 which is also of generally cylindrical hollow form. The needle holder 53 has a hollow needle 54 received fixedly or detachably within one end, the needle 54 having a hollow tip 55 which is of sufficient internal diameter to accommodate the implant 56 therein. Furthermore an elongate plunger 57 extends axially within the needle holder 53 and within the needle 54 so as to contact the implant 56 within the needle tip 55. The plunger 57 is slidable relative to the handgrip 52 within a bore 64 and is integrally formed with a slide part 58 having two guide rods 59 extending through two guide bores 60 in the handgrip 52, as best seen in Figure 2. The guide rods 59 are in turn connected to a depth guide in the form of an end cap 61.

In addition the implanter 50 incorporates a detachable needle tip protector 70, as shown in broken lines in Figure 1, in the form of a transparent plastic sleeve which is an interference fit on the needle 54 and which has an end portion 71 extending beyond the needle tip 55 when fitted to the needle 54. As best seen in the detailed view of the needle tip 55 and the end of the needle tip protector 70 as shown in Figure 3, the end portion 71 incorporates a guide passage 72 extending from the end of the needle tip protector 70 to the end of the sleeve bore 73 for receiving the needle 54. The guide passage 72 has an internal diameter corresponding to the internal diameter of the needle 54 to enable the implant 56 to be introduced into the needle tip 55 by way of the guide passage 72. Furthermore the sleeve bore 73 has an end part 76 which is shaped to conform to the shape of the needle tip 55 so that the needle 54 fits fully into the protector 70 only when correctly orientated relative to the protector, and so that the guide passage 72 and the internal bore of the needle 54 form a continuous passage through which the implant 56 can be introduced without damage. By using a locator on the protector 70, therefore, it is possible to achieve the required needle orientation very simply during assembly.

A loading member 74 having a shaped end 75, is also provided for carefully pushing the implant 56 into the needle tip 54, as shown in broken lines in Figure 1. The shaped end 75 of the loading member 74 has an outer diameter approximately equal to the inner diameter of the guide passage 72 and the inner bore of the needle 54 so as to allow the shaped end 75 to penetrate the guide passage 72 and the needle bore.

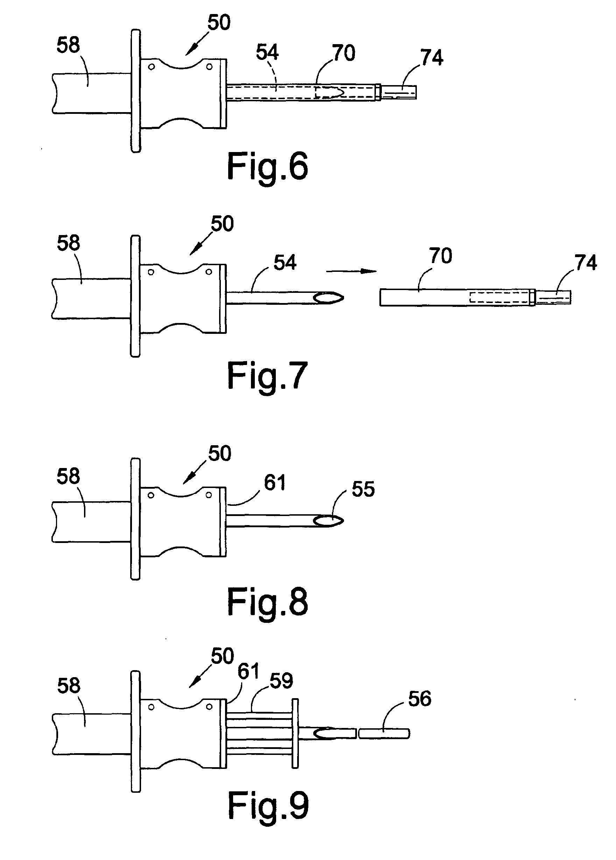

Before the implanter 50 can be used to perform an implant under an animal's skin, it is necessary for the implant 56 to be loaded into the needle tip 55. To this end the implanter 50 is supplied as a unit with the needle tip protector 70 fitted over the needle 54, together with a separate loading member 74, as shown in Figure 4. The implant 56 is loaded into the needle tip 55 by placing the implant 56 in the guide passage 72 in the end of the needle tip protector 70, as shown in Figure 5. The implant 56 is then pushed by means of the loading member 74 from the guide passage 72 fully into the needle tip 55, as shown in Figure 6. If required the complete implanter incorporating the fitted implant can be packaged and sterilised, and supplied in this form to the user. The loading member 74 is a tight fit within the guide passage 72 when in the fully inserted position shown in Figure 5, and remains in place within the guide passage 72. The loading member 74 serves to block the guide passage 72 to prevent contamination from reaching the needle 54 and also to prevent any risk of the implant 56 falling out of the implanter 50 before implantation can be effected.

In use of the implanter 50 to introduce the implant 56 under an animal's skin, the needle tip protector 70 is first removed from the needle 54 as shown in Figure 6. The implanter 50 is then held by the user by its handgrip 52, and the needle tip 55, with the implant 56 received therein, is caused to puncture the animal's skin. The operation of the implanter 50 is similar to the operation of a syringe to deliver an injection. The needle tip 55 is inserted to a depth of typically 30 mm whilst exerting pressure on the slide part 58 in a direction towards the animal, as shown in Figure 8. When the needle tip 55 reaches the required depth as indicated by the end cap 61 contacting the surface of the skin, the user pulls the handgrip 52 in a direction away from the animal whilst still pushing the slide part 58 towards the animal so as to cause the needle tip 55 to be withdrawn leaving the implant 56 in position within the animal, as shown in Figure 9. When the handgrip 52 has been drawn back to a sufficient extent the needle tip 55 is shielded by the end cap 61 after being withdrawn from the animal's skin, and is locked in position by engagement of ears 62 on the end of the slide part 58 within recesses 63 in the handgrip 52, thus preventing the user being accidentally injured by being stuck with the needle 54 and enabling the implanter to be safely disposed of.

The above-described implanter 50 serves the intended function of placing the implant at the required depth under the animal's skin without any special measures having to be taken. Furthermore the plunger 57 is provided with an extension in the form of a thin tailpiece 11, as best seen in Figure 1, which lies between the implant 56 and the inside wall of the needle 54 and which serves to jam the implant 56 within the needle tip 55 until expulsion of the implant 56 from the needle tip 55 is effected by relative movement of the needle 54 and the plunger 57. Whilst such an implanter is particularly suitable for placement of an electronic tag implant, it can also be used with other types of implant, such as a slow release pharmaceutical implant for the release of hormonal or therapeutic agents, for example.

## Claims

1. An implanter for introducing an implant under the skin of a human or animal body, the implanter (50) comprising a needle holder (53) for a hollow needle (54) having a tip (55) within which the implant (56) is accommodated and by means of which the implant is introduced under the skin, and a plunger (57) for relative movement within the needle, **characterised in that** the implanter (50) incorporates needle tip protection means (70) for shielding the needle tip (55) prior to use and having guide means (72) for enabling the implant (56) to be loaded within the needle tip (55) whilst the needle tip (55) is shielded by the needle tip protection means (70).

2. An implanter according to claim 1, wherein the needle tip protection means (70) incorporates a portion (71) which extends beyond the needle tip (55) when in a shielding position, and the guide means comprises a guide passage (72) in said portion (71) through which the implant (56) is loadable.

3. An implanter according to claim 1 or 2, wherein the needle tip protection means (70) is detachable from the implanter (50) to enable the implant (56) to be introduced under the skin by the needle (54).

4. An implanter according to claim 1, 2 or 3, wherein the needle tip protection means comprises sleeve means (70) which surrounds at least an end portion of the needle (54) when in a shielding position.

5. An implanter according to claim 4, wherein the sleeve means (70) has an inner bore (73) for receiving the needle (54) having an end part (76) which is adapted to the shape of the needle tip (55) to ensure a required relative orientation between the sleeve means (70) and the needle (54).

6. An implanter according to any preceding claim, wherein a loading member (74) is provided for pushing the implant (56) into the needle tip (55) by way of the guide means (72).

7. An implanter according to any preceding claim, wherein a depth guide (61) is provided for contacting the surface of the skin during introduction of the needle tip (55) under the skin and for effecting relative movement between the needle (54) and the plunger (57) when the needle tip (55) has been introduced under the skin to a required depth in order to cause the implant (56) to be left at the required depth when the needle tip (55) is withdrawn.

8. An implanter according to any preceding claim, wherein needle shielding means (61) is provided for shielding the needle tip (55) when it has been withdrawn from the skin.

9. An implanter according to claims 7 and 8, wherein the needle shielding means is constituted by a part of the depth guide (61) into which the needle tip (55) is retractable after completion of implantation.

10. An implanter according to claim 8 or 9, wherein locking means (62, 63) is provided to hold the needle (54) in a retracted position in which the needle tip (55) is shielded by the needle shielding means (61) and accidental exposure of the needle tip (55) is prevented.

11. An implanter according to claim 10, wherein the locking means comprises a latch (62, 63) which serves to couple together the needle shielding means (61) and the needle holder (53) in order to prevent relative movement between the needle (54) and the needle shielding means (61) in such a manner as to expose the needle tip (55).

12. An implanter according to any preceding claim, wherein the implanter (50) incorporates a substantially cylindrical handgrip (52) from which the needle (54) axially extends and which is adapted to be grasped by the user during introduction of the implant (56).

13. An implanter according to any preceding claim, **characterised in that** a spring is provided to effect automatic retraction of the needle (54) relative to the plunger (57) to expel the implant (56) from the needle (54) when the needle tip (55) has been introduced under the skin to the required depth.
